# EUROPÄISCHE PATENTSCHRIFT

(11) **EP 4 396 034 B1**
(45) Veröffentlichungstag und Bekanntmachung des Hinweises auf die Patenterteilung: **08.07.2026**
(21) Anmeldenummer: 22773220.3
(22) Anmeldetag: 05.09.2022
(51) Int. Cl.: B60N 2/70, B60N 2/90, B29C 44/12

(54) **GEHÄUSE FÜR EINE VIBRATIONSEINHEIT, VIBRATIONSEINHEIT, SITZPOLSTER UND VERFAHREN ZUM HERSTELLEN EINES SITZPOLSTERS**
HOUSING FOR A VIBRATION UNIT, VIBRATION UNIT, SEAT CUSHION AND METHOD FOR PRODUCING A SEAT CUSHION
BOÎTIER POUR UNE UNITÉ DE VIBRATION, UNITÉ DE VIBRATION, COUSSIN DE SIÈGE ET PROCÉDÉ DE PRODUCTION D'UN COUSSIN DE SIÈGE

(30) Priorität: 03.09.2021 DE 102021122823
(43) Veröffentlichungstag der Anmeldung: 10.07.2024
(73) Patentinhaber: Ford Global Technologies, LLC, Dearborn, MI 48126-2701 (US); Ford-Werke GmbH, 50735 Köln (DE)
(72) Erfinder: CONRADY, Heiko, 40625 Düsseldorf (DE); MEYER, Sebastian, 40789 Monheim (DE)
(74) Vertreter: Markowitz, Markus
(86) Internationale Anmeldenummer: PCT/EP2022/074617
(87) Internationale Veröffentlichungsnummer: WO 2023/031458

(56) Entgegenhaltungen:
- EP-B1- 2 870 024
- FR-B1- 2 866 151
- JP-A- 2017 213 243

## Beschreibung

Die Erfindung betrifft ein Gehäuse für eine an einem Sitzpolster eines Fahrzeugsitzes anordbare oder angeordnete Vibrationseinheit zum Erzeugen eines haptisch erfassbaren Signals an dem Fahrzeugsitz. Zudem betrifft die Erfindung eine Vibrationseinheit zum Erzeugen eines haptisch erfassbaren Signals an einem Sitzpolster eines Fahrzeugsitzes, aufweisend wenigstens eine elektrisch ansteuerbare Vibrationseinrichtung zum Erzeugen von Vibrationen. Darüber hinaus betrifft die Erfindung ein Sitzpolster für einen Fahrzeugsitz. Ferner betrifft die Erfindung ein Verfahren zum Herstellen eines Sitzpolsters für einen Fahrzeugsitz, wobei das Sitzpolster durch Aufschäumen und Aushärten eines Kunststoffs innerhalb eines Formwerkzeugs hergestellt wird. Zusätzlich betrifft die Erfindung ein Verfahren zum Bereitstellen eines akustisch und haptisch wahrnehmbaren und somit verbesserten Fahrerlebnisses für einen Insassen, insbesondere Fahrer.

Die schwingungstechnische und akustische Optimierung moderner Fahrzeuge, insbesondere batterieelektrischer Fahrzeuge, und die damit verbundene Reduzierung von Vibrationen und Geräuschen, die von den Fahrzeuginsassen wahrgenommen werden, hat dazu geführt, dass das eigentliche Fahrerlebnis in den Hintergrund tritt. Es besteht daher ein Bedarf, das Fahrerlebnis wahrnehmbarer zu gestalten und somit zu verbessern.

Es ist bekannt, in einem Fahrzeug eine Vibrationseinheit an einem Fahrzeugbauteil zu verbauen, dass den Körper einer in dem Fahrzeug sitzenden Person berührt. Durch die Aktivierung einer solchen Vibrationseinheit erzeugt diese Vibrationen an dem Fahrzeugbauteil, die von der Person haptisch wahrnehmbar sind, beispielsweise um der Person eine Warnung oder einen Sicherheitshinweis zu geben.

Aus der US 5 022 384 A ist eine Vibrationsquelle für ein Sitzmöbel bekannt, die dem Benutzer des Sitzes einen therapeutischen Nutzen bietet. Die Vibrationsquelle kann beispielsweise in einem Massagesitz eines Fahrzeuges verwendet werden, um von langen Reisen verursachte Verspannungen zu lösen. Allerdings ist diese Vibrationsquelle nicht geeignet, ein Fahrerlebnis eines Insassen eines Fahrzeuges zu verbessern. JP 2017 213243 A zeigt weiter einen Fahrzeugsitz mit einer Vibrationseinheit aus dem Stand der Technik.

Der Erfindung liegt die Aufgabe zugrunde, ein Fahrerlebnis eines Insassen, insbesondere Fahrers, eines Fahrzeugs zu verbessern.

Erfindungsgemäß wird die Aufgabe durch ein Gehäuse mit den Merkmalen des Anspruchs 1 gelöst.

Es ist darauf hinzuweisen, dass die in der nachfolgenden Beschreibung einzeln aufgeführten Merkmale sowie Maßnahmen in beliebiger technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Mit der Erfindung kann eine Vibrationseinheit mit einer kantenfreien bzw. glatten, beispielsweise vollständig abgerundeten, Oberfläche zur Verfügung gestellt werden, wodurch die Vibrationseinheit in ein Sitzpolster eines Fahrzeugsitzes integrierbar ist, ohne dass die Vibrationseinheit von einer auf dem Fahrzeugsitz sitzenden Person als störend empfunden wird und ohne dass das Sitzpolster durch Relativbewegungen zwischen dem Sitzpolster und der Vibrationseinheit beschädigt wird.

Zudem kann aufgrund der Erfindung die Vibrationseinheit zum gleichzeitigen Erzeugen eines Vibrationssignals und eines akustischen Signals verwendet werden, da eine innerhalb des Gehäuses angeordnete Vibrationseinrichtung während der Erzeugung von Vibrationen gleichzeitig auch Schallwellen erzeugt, die durch die wenigstens eine Gehäuseöffnung aus der Vibrationseinheit austreten können, um für eine auf dem Fahrzeugsitz sitzende Person akustisch wahrnehmbar zu werden. Beispielsweise kann dadurch ein Motorstart simuliert werden. Verschiedene Klangdaten können verwendet werden, um gewünschte Vibrationen und Klänge zu erzeugen. Die Klangdaten sind in einem Steuergerät, beispielsweise in einer zentralen Steuereinheit oder einer dezentralen Steuereinheit hinterlegt und können individuell oder vorgebbar abrufbar sein. So kann beispielsweise einem Fahrer eines batterieelektrischen oder anders angetriebenen Fahrzeugs das Fahrgefühl und der Klang eines Fahrzeugs mit Verbrennungsmotor mit den diesem innenwohnenden typischen Eigenschaften vermittelt werden.

Die Gehäuseöffnung kann beispielsweise als kreisförmige oder polygonale Durchbrechung an dem Gehäuse ausgebildet sein. Die Gehäuseöffnung kann an einer Seite des Gehäuses angeordnet sein, die bei einer bestimmungsgemäßen Anordnung der Vibrationseinheit an dem Fahrzeugsitz einer Sitzfläche des Fahrzeugsitzes zugewandt angeordnet ist. Das Gehäuse kann auch zwei oder mehr solcher Gehäuseöffnungen aufweisen.

Die Erfindung bietet somit einem Insassen, insbesondere Fahrer, eines Fahrzeugs ein akustisch und haptisch wahrnehmbares und somit verbessertes Fahrerlebnis, und das unter Verwendung von lediglich einem einzigen Gerät, nämlich der Vibrationseinheit, die sich an oder in dem Fahrzeugsitz und daher in der Nähe des Körpers des Insassen befindet. Wenn eine Vibrationseinheit benachbart zu Verstärkungselementen, z.B. Verstärkungsdrähten des Fahrzeugsitzes angeordnet ist, wird die Übertragung von Vibrationen und Klang durch den strukturgetragenen Klang noch besser.

Das Gehäuse kann teilweise oder vollständig aus einem Kunststoff, insbesondere einem faserverstärkten Kunststoff, einem Metall oder einer Metalllegierung gebildet sein. Das Gehäuse kann wenigstens zwei miteinander verbundene Gehäuseteile aufweisen. Innerhalb des Gehäuses ist ein Aufnahmeraum zur vollständigen Aufnahme einer Vibrationseinrichtung zum Erzeugen von Vibrationen ausgebildet.

Erfindungsgemäß ist das Gehäuse als triaxiales Ellipsoid oder nierenförmig ausgebildet. Diese Ausgestaltung des Gehäuses geht mit einer kantenfreien bzw. vollständig abgerundeten Oberfläche des Gehäuses einher.

Die obige Erfindung wird zudem durch eine Vibrationseinheit mit den Merkmalen des Anspruchs 3 gelöst, die ein Gehäuse nach einer der oben genannten Ausgestaltungen oder einer Kombination von wenigstens zwei dieser Ausgestaltungen miteinander aufweist, in dem die Vibrationseinrichtung vollständig aufgenommen ist.

Mit der Vibrationseinheit sind die oben mit Bezug auf das Gehäuse genannten Vorteile entsprechend verbunden. Die elektrisch ansteuerbare Vibrationseinrichtung kann beispielsweise wenigstens einen Elektroantrieb und einen damit antreibbaren Exzenter aufweisen. Der elektrisch ansteuerbaren Vibrationseinrichtung kann aber auch jedes andere Wirkprinzip zur Erzeugung von Vibrationen und Schallwellen zugrunde liegen. Die elektrisch ansteuerbare Vibrationseinrichtung kann also beispielsweise auch wenigstens einen piezoelektrischen oder einen elektromagnetischen Aktuator aufweisen. Die obige Erfindung wird zudem durch ein Sitzpolster mit den Merkmalen des Anspruchs 4 gelöst, das wenigstens eine oben genannte Vibrationseinheit aufweist, die vollständig innerhalb des Sitzpolsters eingebettet ist.

Mit dem Sitzpolster sind die oben mit Bezug auf das Gehäuse genannten Vorteile entsprechend verbunden. Die Platzierung der Vibrationseinheit im Inneren des Sitzpolsters bzw. eines Polsterabschnitts davon, das im Übrigen beispielsweise aus einem Schaumstoff gebildet sein kann, ist eine sehr gute technische Lösung zur Anordnung der Vibrationseinheit an dem Sitzpolster.

Die obige Erfindung wird zudem durch ein Verfahren mit den Merkmalen des Anspruchs 6 gelöst, gemäß dem innerhalb des Formwerkzeug wenigstens eine Kunststofftasche zur Aufnahme einer Vibrationseinheit, insbesondere nach einer der oben genannten Ausgestaltungen oder einer Kombination von wenigstens zwei dieser Ausgestaltungen miteinander, derart angeordnet ist, dass sie größtenteils in das Sitzpolster eingebettet ist und ihre Taschenöffnung in eine Außenfläche des Sitzpolsters integriert oder außerhalb des Sitzpolsters angeordnet ist. Das Sitzpolster kann ein Teil einer Rückenlehne oder einer Sitzfläche des Fahrzeugsitzes sein.

Mittels der Kunststofftasche kann innerhalb des Sitzpolsters ein über die Taschenöffnung, die an der Oberfläche des Sitzpolsters oder außerhalb des Sitzpolsters angeordnet ist, zugänglicher Hohlraum zur vollständigen Aufnahme der Vibrationseinheit ausgebildet werden. Die Platzierung einer herkömmlichen Vibrationseinheit im Inneren eines herkömmlichen Sitzpolsters ist hingegen aufgrund des herkömmlichen Aufschäumprozesses nicht möglich.

Gemäß einer vorteilhaften Ausgestaltung wird die Kunststofftasche während des Aufschäumens und Aushärtens des Kunststoffs in einem aufgeblasenen Zustand gehalten. Hierdurch wird innerhalb des Sitzpolsters ein Hohlraum ausgebildet, in den die Vibrationseinheit beispielsweise formschlüssig einfügbar ist.

Gemäß einer weiteren vorteilhaften Ausgestaltung wird die Kunststofftasche durch zwei Kunststofffolien gebildet, die unter Belassung einer wiederverschließbaren Taschenöffnung randseitig stoffschlüssig miteinander verbunden sind, wobei ein thermisches Verschweißen der beiden Kunststofffolien durchgeführt werden könnte, was aber nicht beschränkend sein soll. Hierdurch kann die Kunststofftasche auf einfache Art und Weise sowie kostengünstig hergestellt werden. Zudem kann die Formgebung der Kunststofftasche auf einfache Art und Weise an die jeweilige Formgebung der Vibrationseinheit angepasst werden.

Gemäß einer weiteren vorteilhaften Ausgestaltung wird an der Kunststofftasche wenigstens eine Durchbrechung und/oder wenigstens eine Lasche mit wenigstens einer Durchbrechung ausgebildet, wobei die jeweilige Durchbrechung von einem Abschnitt des Sitzpolsters durchdrungen wird. Die Durchbrechung und/oder Lasche ist in idealer Ausgestaltung wiederverschließbar ausgeführt, wobei diese so mediendicht ist, dass diese bei dem Aufschäumen und Aushärten des Kunststoffs im aufgeblasenen Zustand verbleibt. Wird die Durchbrechung und/oder Lasche nach dem Aufschäumen und Aushärten geöffnet, um die Vibrationseinheit in den geschaffenen Hohlraum einzuführen, verbleibt der Hohlraum in seiner durch die Kunststofftasche gebildeten Dimension, so dass ein leichtes Einbringen der Vibrationseinheit gesichert ist. Nach dem Einbringen wird die Kunststofftasche wieder verschlossen. Mediendichte Wiederverschlusselemente sind allgemein bekannt, weswegen hier nicht weiter darauf eingegangen wird.

Weitere vorteilhafte Ausgestaltungen der Erfindung sind in den Unteransprüchen und der folgenden Figurenbeschreibung offenbart. Es zeigt
- Fig. 1a-c: schematische Darstellungen eines Ausführungsbeispiels für ein erfindungsgemäßes Sitzpolster in drei verschiedenen Zuständen,
- Fig. 2: eine schematische Darstellung eines weiteren Ausführungsbeispiels für ein erfindungsgemäßes Sitzpolster und
- Fig. 3: eine schematische Darstellung eines Ausführungsbeispiels für einen erfindungsgemäßen Fahrzeugsitz.

In den unterschiedlichen Figuren sind gleiche Teile stets mit denselben Bezugszeichen versehen, weswegen diese in der Regel auch nur einmal beschrieben werden.

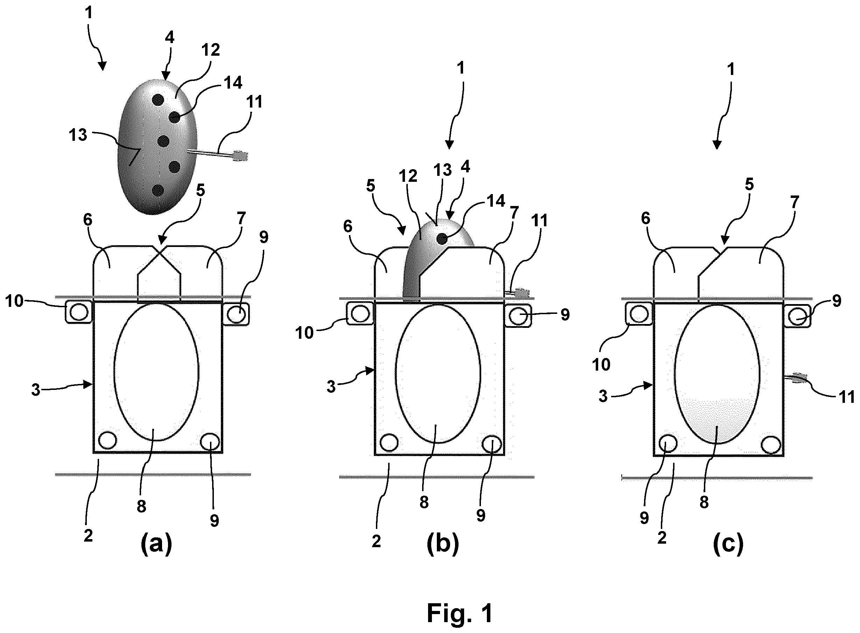

Die Figuren 1a-c zeigen schematische Darstellungen eines Ausführungsbeispiels für ein erfindungsgemäßes Sitzpolster 1 für einen nicht gezeigten Fahrzeugsitz in drei verschiedenen Zuständen.

Das Sitzpolster 1 weist einen Polsterabschnitt 2 auf, der durch Aufschäumen und Aushärten eines Kunststoffs innerhalb eines nicht gezeigten Formwerkzeugs hergestellt worden ist. Innerhalb des Polsterabschnitts 2 ist eine Kunststofftasche 3 zur Aufnahme einer Vibrationseinheit 4 des Sitzpolsters 1 teilweise angeordnet, indem die Kunststofftasche 3 innerhalb des Formwerkzeug derart angeordnet worden ist, dass sie größtenteils in den Polsterabschnitt 2 eingebettet ist und ihre Taschenöffnung 5 außerhalb des Polsterabschnitts 2 angeordnet ist. Dabei kann die Kunststofftasche 3 während des Aufschäumens und Aushärtens des Kunststoffs in einem aufgeblasenen Zustand gehalten worden sein.

Die Kunststofftasche 3 ist durch zwei Kunststofffolien 6 und 7 gebildet, die unter Belassung der Taschenöffnung 5 randseitig derart stoffschlüssig miteinander verbunden sind, dass mittig ein Aufnahmeraum 8 für die Vibrationseinheit 4 vorhanden ist. Beispielsweise können die beiden Kunststofffolien 6 und 7 randseitig thermisch verschweißt sein. Ist die Vibrationseinheit 4 zwangsläufig vollständig innerhalb des Aufnahmeraums 8 angeordnet, ist sie gleichzeitig vollständig innerhalb des Polsterabschnitts 2 eingebettet.

An der Kunststofftasche 3 sind zwei Durchbrechungen 9 und zwei Laschen 10 mit jeweils einer Durchbrechung 9 ausgebildet, wobei die jeweilige Durchbrechung 9 von einem Abschnitt des Polsterabschnitts 2 durchdrungen ist.

Die Vibrationseinheit 4 zum Erzeugen eines haptisch erfassbaren Signals an dem Sitzpolster 1 weist eine nicht gezeigte, elektrisch ansteuerbare Vibrationseinrichtung zum Erzeugen von Vibrationen auf. Zudem weist die Vibrationseinheit 4 eine Kabelverbindung 11 auf, über die die Vibrationseinrichtung elektrisch ansteuerbar ist. Denkbar ist auch eine kabellose Ansteuerung.

Des Weiteren weist die Vibrationseinheit 4 ein Gehäuse 12 auf, in dem die Vibrationseinrichtung vollständig aufgenommen ist. Das Gehäuse 12 weist eine kantenfreie Oberfläche 13 und fünf in einem W-förmigen Muster angeordnete, für Schallwellen durchlässige Gehäuseöffnungen 14 auf. Das Gehäuse 12 ist als triaxiales Ellipsoid ausgebildet.

In dem in Fig. 1a gezeigten Zustand befindet sich die Vibrationseinheit 4 außerhalb der Kunststofftasche 3 bzw. des Polsterabschnitts 2. In dem in Fig. 1b gezeigten Zustand ist die Vibrationseinheit 4 bereits teilweise in die Kunststofftasche 3 eingefügt. In dem in Fig. 1c gezeigten Zustand ist die Vibrationseinheit 4 vollständig in die Kunststofftasche 3 bzw. deren Aufnahmeraum 8 eingefügt.

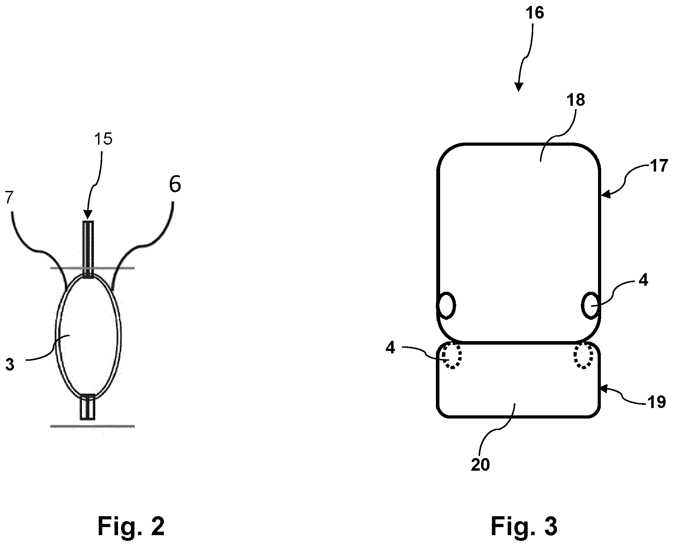

In Figur 2 ist gezeigt, wie die Kunststofftasche 3 nach dem Aufschäumen und Aushärten des Kunststoffs beispielhaft an die Ausgestaltung des Gehäuses 12 angepasst ist. Die Kunststofftasche 3 ist in idealer Ausgestaltung an ihrer Durchbrechung und/oder Lasche 15, also an der Taschenöffnung 5 wiederverschließbar mediendicht ausgeführt, so dass diese bei dem Aufschäumen und Aushärten des Kunststoffs den Hohlraum für das Gehäuse 12 bildet. Zum Einführen des Gehäuses 12 mit der Kabelverbindung 11 wird die Kunststofftasche 3 durch Öffnen der wiederverschließbaren Lasche, also der Taschenöffnung 5 zugänglich, so dass das Gehäuse 12, also die Vibrationseinheit 4 leicht in den Hohlraum, also in die Kunststofftasche 3 eingeführt werden kann. Danach wird die Kunststofftasche 3 wieder verschlossen, wobei die Kabelverbindung 11 zumindest zur Energieversorgung und/oder zur Datenverbindung herausgeführt ist. Bei dem dargestellten Ausführungsbeispiel ist die Kabelverbindung 11 seitlich, in der Zeichnungsebene rechts aus dem Gehäuse 12 und der Kunststofftasche 3 herausgeführt, so dass die wiederverschließbare Taschenöffnung 5 in idealer Ausgestaltung an die Positionierung der Kabelverbindung 11 und die Ausgestaltung des Gehäuses 12 angepasst ist.

Fig. 3 zeigt eine schematische Darstellung eines Ausführungsbeispiels für einen erfindungsgemäßen Fahrzeugsitz 16 für ein nicht gezeigten Landfahrzeug.

Der Fahrzeugsitz 16 weist eine Rückenlehne 17 mit einem Sitzpolster 18 und eine Sitzfläche 19 mit einem Sitzpolster 20 auf. **In** das Sitzpolster 18 sind seitlich und in einem unteren Bereich zwei Vibrationseinheiten 4 eingebettet. Die Vibrationseinheiten 4 können entsprechend dem in den Figuren. 1a-c gezeigten Ausführungsbeispiel ausgebildet sein. Alternativ oder additiv können in das Sitzpolster 20 der Sitzfläche 19 seitlich und in einem hinteren, also rückenlehnennahen Bereich zwei Vibrationseinheiten 4 eingebettet sein, was in Fig. 3 durch gestrichelte Linien angedeutet ist.

### Bezugszeichenliste

- 1: Sitzpolster
- 2: Polsterabschnitt
- 3: Kunststofftasche
- 4: Vibrationseinheit
- 5: Taschenöffnung
- 6: Kunststofffolie
- 7: Kunststofffolie
- 8: Aufnahmeraum von 3
- 9: Durchbrechung
- 10: Lasche von 3
- 11: Kabelverbindung
- 12: Gehäuse
- 13: Oberfläche von 12
- 14: Gehäuseöffnung
- 15: Durchbrechung / Lasche
- 16: Fahrzeugsitz
- 17: Rückenlehne
- 18: Sitzpolster von 17
- 19: Sitzfläche
- 20: Sitzpolster von 19

## Patentansprüche

1. Gehäuse (12) für eine an einem Sitzpolster (1) eines Fahrzeugsitzes (16) anordbare Vibrationseinheit (4) zum gleichzeitigen Erzeugen eines Vibrationssignals und eines akustischen Signals, wobei das Gehäuse (12) eine vollständig abgerundete Oberfläche (13) und wenigstens eine für Schallwellen durchlässige Gehäuseöffnung (14) aufweist,
**dadurch gekennzeichnet, dass**
das Gehäuse (12) als triaxiales Ellipsoid oder nierenförmig ausgebildet ist.

2. Gehäuse (12) nach Anspruch 1
**dadurch gekennzeichnet, dass**
die Gehäuseöffnung (14) an einer Seite des Gehäuses (12) angeordnet ist, die bei einer bestimmungsgemäßen Anordnung der Vibrationseinheit (4) an dem Fahrzeugsitz (16) einer Sitzfläche (19) des Fahrzeugsitzes (16) zugewandt angeordnet ist

3. Vibrationseinheit (4) zum gleichzeitigen Erzeugen eines Vibrationssignals und eines akustischen Signals, aufweisend wenigstens eine elektrisch ansteuerbare Vibrationseinrichtung zum Erzeugen von Vibrationen,
**gekennzeichnet durch**
ein Gehäuse (12) nach einem der Ansprüche 1 oder 2, in dem die Vibrationseinrichtung vollständig aufgenommen ist.

4. Sitzpolster (1) für einen Fahrzeugsitz (16), innerhalb dem wenigstens eine Vibrationseinheit (4) nach Anspruch 3 vollständig eingebettet ist.

5. Sitzpolster (1) nach Anspruch 4, bei dem eine Vibrationseinheit benachbart zu
Verstärkungselementen des Fahrzeugsitzes angeordnet ist, wenn das Sitzpolster sich im montierten Zustand befindet.

6. Verfahren zum Herstellen eines Sitzpolsters (1) nach einem der Ansprüche 4 oder 5, wobei das Sitzpolster (1) durch Aufschäumen und Aushärten eines Kunststoffs innerhalb eines Formwerkzeugs hergestellt wird,
**dadurch gekennzeichnet, dass**
innerhalb des Formwerkzeug wenigstens eine Kunststofftasche (3) zur Aufnahme einer Vibrationseinheit (4) derart angeordnet ist, dass sie größtenteils in das Sitzpolster (1) eingebettet ist und ihre Taschenöffnung (5) in eine Außenfläche des Sitzpolsters (1) integriert oder außerhalb des Sitzpolsters (1) angeordnet ist.

7. Verfahren nach Anspruch 6,
**dadurch gekennzeichnet, dass**
die Kunststofftasche (3) während des Aufschäumens und Aushärtens des Kunststoffs in einem aufgeblasenen Zustand gehalten wird.

8. Verfahren nach Anspruch 6 oder 7,
**dadurch gekennzeichnet, dass**
die Kunststofftasche (3) durch zwei Kunststofffolien (6, 7) gebildet wird, die unter Belassung der Taschenöffnung (5) randseitig stoffschlüssig miteinander verbunden sind.

9. Verfahren nach einem der Ansprüche 6 bis 8,
**dadurch gekennzeichnet, dass**
an der Kunststofftasche (3) wenigstens eine Durchbrechung (9) und/oder wenigstens eine Lasche (10) mit wenigstens einer Durchbrechung (9) ausgebildet wird, wobei die jeweilige Durchbrechung (9) von einem Abschnitt des Sitzpolsters (1) durchdrungen wird.

10. Verfahren zum Bereitstellen eines akustisch und haptisch wahrnehmbaren und somit verbesserten Fahrerlebnisses für einen Insassen, insbesondere Fahrer, unter Verwendung einer Vibrationseinheit (4) nach Anspruch 3, die sich an oder in einem Fahrzeugsitz befindet, aufweisend die Schritte:
- Hinterlegen von Klangdaten in einem Steuergerät
- Abrufen der Klangdaten
- Verwenden der Klangdaten, um gewünschte Vibrationen und Klänge zu erzeugen
- Verwenden der Vibrationseinheit zum gleichzeitigen Erzeugen eines Vibrationssignals und eines akustischen Signals, indem die innerhalb des Gehäuses (12) angeordnete Vibrationseinrichtung während der Erzeugung von Vibrationen gleichzeitig auch Schallwellen erzeugt, die durch die wenigstens eine Gehäuseöffnung (14) aus der Vibrationseinheit (4) austreten können

## Claims

1. Housing (12) for a vibration unit (4) which can be arranged on a seat cushion (1) of a vehicle seat (16) and is intended to simultaneously generate a vibration signal and an acoustic signal, wherein the housing (12) has a completely rounded surface (13) and at least one housing opening (14), which is permeable to sound waves,
**characterized in that**
the housing (12) is designed as a triaxial ellipsoid or is kidney-shaped.

2. Housing (12) according to Claim 1,
**characterized in that**
the housing opening (14) is arranged on a side of the housing (12) which, in the case of an intended arrangement of the vibration unit (4) on the vehicle seat (16), is arranged facing a seat bottom (19) of the vehicle seat (16).

3. Vibration unit (4) for simultaneously generating a vibration signal and an acoustic signal, having at least one electrically controllable vibration device for generating vibrations,
**characterized by**
a housing (12) according to Claim 1 or 2, in which the vibration device is completely accommodated.

4. Seat cushion (1) for a vehicle seat (16), within which at least one vibration unit (4) according to Claim 3 is completely embedded.

5. Seat cushion (1) according to Claim 4, in which a vibration unit is arranged adjacent to reinforcing elements of the vehicle seat when the seat cushion is in the mounted state.

6. Method for producing a seat cushion (1) according to either of Claims 4 and 5, wherein the seat cushion (1) is produced by foaming and curing a plastic within a moulding tool,
**characterized in that**
at least one plastic pocket (3) for accommodating a vibration unit (4) is arranged within the moulding tool in such a way that it is largely embedded in the seat cushion (1) and its pocket opening (5) is integrated into an outer surface of the seat cushion (1) or is arranged outside the seat cushion (1).

7. Method according to Claim 6,
**characterized in that**
the plastic pocket (3) is kept in an inflated state during foaming and curing of the plastic.

8. Method according to Claim 6 or 7,
**characterized in that**
the plastic pocket (3) is formed by two plastic films (6, 7), which are connected to one another in a materially bonded manner at the edge while leaving the pocket opening (5).

9. Method according to any of Claims 6 to 8,
**characterized in that**
at least one aperture (9) and/or at least one tab (10) with at least one aperture (9) are/is formed on the plastic pocket (3), wherein a section of the seat cushion (1) passes through the respective aperture (9).

10. Method for providing an acoustically and haptically perceptible and thus improved driving experience for an occupant, in particular driver, using a vibration unit (4) according to Claim 3, which is located on or in a vehicle seat, comprising the steps:
- storing sound data in a control unit
- retrieving the sound data
- using the sound data to produce desired vibrations and sounds
- using the vibration unit for the simultaneous generation of a vibration signal and an acoustic signal, in that, during the generation of vibrations, the vibration device arranged within the housing (12) simultaneously also generates sound waves and these can emerge from the vibration unit (4) through the at least one housing opening (14)

## Revendications

1. Boîtier (12) pour une unité de vibration (4) susceptible d'être disposée sur un coussin de siège (1) d'un siège de véhicule (16) pour générer simultanément un signal de vibration et un signal acoustique, le boîtier (12) présentant une surface (13) entièrement arrondie et au moins une ouverture de boîtier (14) perméable aux ondes sonores,
**caractérisé en ce que**
le boîtier (12) est réalisé sous forme d'ellipsoïde triaxial ou en forme de rein.

2. Boîtier (12) selon la revendication 1,
**caractérisé en ce que**
l'ouverture de boîtier (14) est disposée sur un côté du boîtier (12) qui, lors d'un agencement conforme de l'unité de vibration (4) sur le siège de véhicule (16), est disposé face à une surface d'assise (19) du siège de véhicule (16).

3. Unité de vibration (4) pour générer simultanément un signal de vibration et un signal acoustique, comportant au moins un dispositif de vibration pouvant être commandé électriquement pour générer des vibrations, **caractérisé par**
un boîtier (12) selon l'une quelconque des revendications 1 ou 2, dans lequel le dispositif de vibration est intégralement incorporé.

4. Coussin de siège (1) pour un siège de véhicule (16), à l'intérieur duquel au moins une unité de vibration (4) selon la revendication 3 est intégralement incorporée.

5. Coussin de siège (1) selon la revendication 4, dans lequel une unité de vibration adjacente à des éléments de renforcement du siège de véhicule est disposée lorsque le coussin de siège se trouve à l'état monté.

6. Procédé de fabrication d'un coussin de siège (1) selon l'une quelconque des revendications 4 ou 5, dans lequel le coussin de siège (1) est fabriqué par moussage et durcissement d'une matière plastique à l'intérieur d'un moule,
**caractérisé en ce que**
au moins une poche en matière plastique (3) destinée à recevoir une unité de vibration (4) est disposée à l'intérieur du moule de telle sorte qu'elle est en grande partie incorporée dans le coussin de siège (1) et que son ouverture de poche (5) est intégrée dans une surface extérieure du coussin de siège (1) ou disposée à l'extérieur du coussin de siège (1).

7. Procédé selon la revendication 6,
**caractérisé en ce que**
la poche en matière plastique (3) est maintenue à l'état gonflé pendant le moussage et le durcissement de la matière plastique.

8. Procédé selon la revendication 6 ou 7,
**caractérisé en ce que**
la poche en matière plastique (3) est formée par deux feuilles de matière plastique (6, 7) qui sont reliées l'une à l'autre par complémentarité de matière sur leurs bords tout en laissant l'ouverture de poche (5).

9. Procédé selon l'une quelconque des revendications 6 à 8,
**caractérisé en ce que**
au moins un ajour (9) et/ou au moins une patte (10) dotée d'au moins un ajour (9) est formé sur la poche en matière plastique (3), l'ajour (9) respectif étant traversé par une partie du coussin de siège (1).

10. Procédé de fourniture d'une expérience de conduite perceptible acoustiquement et haptiquement, et ainsi améliorée, pour un occupant, notamment un conducteur, par utilisation d'une unité de vibration (4) selon la revendication 3, qui est située sur ou dans un siège de véhicule, comprenant les étapes consistant à :
- stocker des données sonores dans une unité de commande
- appeler les données sonores
- utiliser les données sonores pour générer des vibrations et des sons souhaités
- utiliser l'unité de vibration pour générer simultanément un signal de vibration et un signal acoustique, par le fait que le dispositif de vibration disposé à l'intérieur du boîtier (12) génère également, simultanément à la génération de vibrations, des ondes sonores qui peuvent sortir de l'unité de vibration (4) par ladite au moins une ouverture de boîtier (14).
